# FASCICULE DE BREVET EUROPEEN

(11) **EP 2 207 556 B1**
(45) Date de publication et mention de la délivrance du brevet: **04.01.2017**
(21) Numéro de dépôt: 08855581.8
(22) Date de dépôt: 07.11.2008
(51) Int. Cl.: A61K 31/724, A61K 31/05, A61K 31/12, A61P 37/06, A61P 39/06, A01N 1/02

(54) **COMPOSITIONS À BASE DE CYCLODEXTRINES CHARGÉES POSITIVEMENT POUR CONSERVER DES CELLULES, DES TISSUS OU DES ORGANES, ET LEURS UTILISATIONS**
ZUSAMMENSETZUNGEN AUF BASIS POSITIV GELADENER CYCLODEXTRINE ZUR ERHALTUNG VON ZELLEN, GEWEBEN UND ORGANEN, UND ANWENDUNGEN DAVON
COMPOSITIONS BASED ON POSITIVELY-CHARGED CYCLODEXTRINS FOR PRESERVING CELLS, TISSUES AND ORGANS, AND USES THEREOF

(30) Priorité: 08.11.2007 FR 0758881; 12.12.2007 US 954323
(43) Date de publication de la demande: 21.07.2010
(73) Titulaire: Biocydex, 86000 Poitiers (FR); UNIVERSITE DE POITIERS, 86000 Poitiers (FR); Centre Hospitalier Universitaire de Poitiers, 86021 Poitiers Cedex (FR); Institut National Sante Recherche Medicale, 75654 Paris Cedex 13 (FR)
(72) Inventeur: CENATIEMPO, Yves, F-86800 Saint Julien L'ars (FR); BELGSIR, El mustapha, F-86000 Poitiers (FR); RAMNATH, Manilduth, F-86180 Buxerolles (FR); FAVREAU, Frédéric, F-86270 Coussay Les Bois (FR); CARRETIER, Michel, F-86600 Cloue (FR); MAUCO, Gérard, F-86280 Saint Benoit (FR); HAUET, Thierry, F-86550 Mignaloux Beauvoir (FR)
(74) Mandataire: Aquinov
(86) Numéro de dépôt international: PCT/FR2008/052015
(87) Numéro de publication internationale: WO 2009/068798

(56) Documents cités:
- WO-A-93/09790
- WO-A-96/31220
- FR-A- 2 714 067
- US-A- 5 599 659
- US-A- 5 620 961
- EIREF SIMON D ET AL: "Beta-Cyclodextrin prevents cardiac allograft vasculopathy and improves the function and survival of rat cardiac allografts" SURGICAL FORUM, vol. 48, no. 0, 1997, pages 484-486, XP009100992 ISSN: 0071-8041
- WEISZ P B ET AL: "PROTECTION OF ERYTHROCYTES AGAINST HEMOLYTIC AGENTS BY CYCLODEXTRIN POLYSULFATE" BIOCHEMICAL PHARMACOLOGY, PERGAMON, OXFORD, GB, vol. 45, no. 5, 9 mars 1993 (1993-03-09), pages 1011-1016, XP001105544 ISSN: 0006-2952

## Description

La présente invention concerne une composition pour la protection et la préservation d'organes, de tissus ou de cellules.

L'invention se rapporte également aux utilisations de cette composition.

Dans de nombreux domaines, notamment en thérapie cellulaire, tissulaire ou pour la transplantation d'organes, il est primordial de pouvoir conserver correctement et garder viables des cellules, des tissus ou des organes, à l'extérieur d'un organisme.

En effet, les conditions de conservation des cellules, tissus ou organes durant et après leur prélèvement jusqu'à leur greffe, jouent un rôle clé pour leur fonctionnement et leur viabilité à long terme.

On sait notamment que dans le cas de la transplantation d'organes, des lésions tissulaires plus ou moins réversibles apparaissent pendant les périodes d'ischémie froide et chaude et s'aggravent au moment de la reperfusion, pouvant entraîner par la suite une dysfonction chronique des greffons.

Il est donc nécessaire d'utiliser des solutions de préservation capables de maintenir en bon état les cellules vivantes, de les protéger des effets délétères d'un prélèvement et d'une greffe, et de diminuer l'incidence des complications post-opératoires.

De plus, l'ensemble du processus de transplantation étant fréquemment soumis à une contrainte de temps, il est souhaitable que les solutions de préservation permettent une conservation efficace la plus longue possible.

Il existe actuellement un grand nombre de solutions de conservation. On connaît notamment la solution de l'Université du Wisconsin ou UW ou ViaSpan®, considérée comme la solution de référence. D'autres solutions ont également été mises au point depuis une quinzaine d'années comme IGL.-1®,-SCOT®, Celsior® ou encore Custodiol®.

Chacune de ces solutions présente des formulations différentes et des propriétés qui leur sont propres mais aucune ne répond aux exigences précitées et ne procure une qualité réellement satisfaisante.

En outre, il subsiste une pénurie de donneurs, et on se trouve de plus en plus confrontés à des donneurs marginaux dont les organes doivent être conservés de façon optimale.

Il existe donc un besoin pour améliorer les solutions existantes, en particulier pour permettre de mieux conserver une cellule, un tissu ou un organe, pendant plus longtemps, et de restaurer davantage et plus rapidement sa fonction après transplantation.

C'est ce à quoi répond la présente invention qui propose une composition pour protéger et préserver des cellules, des tissus ou des organes, comprenant au moins une cyclodextrine modifiée chargée positivement.

En effet, de façon surprenante, la présence d'une cyclodextrine modifiée chargée positivement présente une très grande efficacité pour protéger et préserver des cellules, des tissus ou des organes.

Préférentiellement, dans la composition selon l'invention la cyclodextrine modifiée chargée positivement est intimement associée à un anti-oxydant.

Par intimement associée, on entend interagissant par tout type de liaison non covalente.

L'anti-oxydant peut-être choisi parmi la vitamine A,B, C ou E, un flavonriide ou un caroténoide tel que le béta-carotène ou le lycopène, ou encore un polyphénol tel que la curcumine.

De manière préférée, l'antioxydant est le resvératrol.

Le resvératrol est une phytoallexine produite par certaines plantes en réponse à une agression par des micro-organismes. La plante productrice la plus connue est la vigne (Vitis vinifera) mais il existe d'autres sources dont la renouée du Japon (Polygonum cuspidatum), plante utilisée dans la pharmacopée traditionnelle asiatique.

Le resvératrol est connu comme anti-oxydant, mais également comme anticancéreux, anti-métastatique, anti-inflammatoire et anti-vieillissement. L'utilisation du resvératrol pour protéger les organes des effets délétères de l'ischémie-reperfusion a été envisagée dans différentes publications mais aucune solution efficace n'a été proposée.

Avantageusement, une composition qui comprend à la fois une cyclodextrine modifiée chargée positivement et un anti-oxydant, en particulier du resvératrol, intimement associés, présente des propriétés remarquables qui permettent de protéger, préserver et/ou conserver efficacement des organes, des cellules ou des tissus ex vivo, c'est-à-dire en dehors d'un organisme vivant.

Enfin, l'invention vise les utilisations en vue de protéger, conserver et/ou préserver des cellules, des tissus ou des organes, d'une composition comprenant au moins une cyclodextrine modifiée chargée positivement.

L'invention est maintenant décrite en détail en regard d'exemples non limitatifs et des figures annexées sur lesquelles :
- la figure 1A est une présentation graphique de résultats relatifs à la reprise de diurèse, en mL. jour⁻¹, mesurée pendant 30 jours après transplantation d'un rein chez le porc, pour différentes solutions de conservation du rein avant transplantation,
- la figure 1B est une présentation graphique de résultats relatifs à la créatinine, en µmol. L⁻¹, mesurée pendant 30 jours après transplantation d'un rein chez le porc, pour différentes solutions de conservation du rein avant transplantation,
- la figure 1C est une présentation graphique de résultats relatifs à la protéinurie en g. jour⁻¹, mesurée pendant 30 jours après transplantation d'un rein chez le porc, pour différentes solutions de conservation du rein avant transplantation,
- la figure 2A est une présentation graphique de résultats relatifs à la reprise de diurèse en mL. jour⁻¹, mesurée pendant 30 jours après transplantation d'un rein chez le porc, pour différentes solutions de conservation du rein avant transplantation,
- la figure 2B est une présentation graphique de résultats relatifs à la créatinine en µmol. L⁻¹, mesurée pendant 30 jours après transplantation d'un rein chez le porc, pour différentes solutions de conservation du rein avant transplantation,
- la figure 2C est une présentation graphique de résultats relatifs à la protéinurie en g. jour⁻¹, mesurée pendant 30 jours après transplantation d'un rein chez le porc, pour différentes solutions de conservation du rein avant transplantation,
- la figure 3 est une présentation graphique de résultats de l'analyse anatomopathologique des surfaces fibrosées de reins greffés chez le porc, 30 jours après la transplantation, pour différentes solutions de conservation du rein avant transplantation, et
- la figure 4 est une présentation graphique de résultats de l'analyse anatomopathologique de l'atrophie tubulaire de reins greffés chez le porc, 30

jours après la transplantation, pour différentes solutions de conservation du rein avant transplantation.

Les cyclodextrines utilisées selon l'invention sont des cyclodextrines modifiées chargées positivement.

Ces cyclodextrines peuvent répondre à la formule : 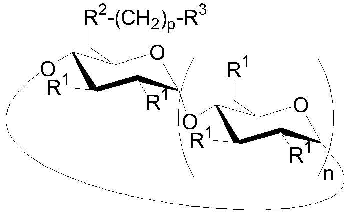 dans laquelle R¹ représente OH ou R²-(CH₂)p-R³ ; R² représente -O- ou -S- ou - NH- ou -NR⁴- ou -N⁺R⁴- ; R³ représente -OH ou -NH₂ ou -NHR⁴ ou -NR⁴ ou -N⁺R4 ; R⁴ représente un groupe alkyle ; n est égal à 5, 6 ou 7, et p est un nombre entier allant de 2 à 10 ; les p, R², R³ et R⁴ pouvant être différents lorsque un ou plusieurs des R¹ représentent R²-(CH₂)p-R³. Lorsque R² et R³ représentent respectivement -NR⁴-, -N⁺R⁴- et NHR⁴, -NR⁴, -N⁺R⁴ avec R⁴ étant un groupe alkyle, celui-ci est de préférence un groupe alkyle inférieur, ayant de 1 à 4 atomes de carbone.

De façon préférée il s'agit de cyclodextrines mono-modifiées.

Encore plus préférentiellement on choisit les cyclodextrines modifiées sur l'hydroxyle primaire et en particulier une 6A-(n-Aminoalkylamino)-6A-desoxy-cyclomatoheptaose telle que la 6A-(3-Aminopropylamino)-6A-desoxy-cyclomatoheptaose (propane diamine-β-CD) ou la 6A-(2-Aminoethylamino)-6A-desoxy-cyclomatoheptaose (éthane diamine-β-CD) décrites dans le brevet FR-2.714.067.

Selon un mode de réalisation particulièrement adapté de l'invention, les cyclodextrines modifiées chargées positivement sont intimement associées à un anti-oxydant.

Les cyclodextrines sont préférentiellement présentes en excès par rapport à l'anti-oxydant. De façon préférée, la composition comprend un ratio molaire anti-oxydant/cyclodextrine compris entre 1/1,5 et 1/150. Encore plus préférentiellement elle comprend un rapport molaire resvératrol/cyclodextrine compris entre 1/80 et 1/120.

La composition selon l'invention peut se présenter sous forme solide, notamment poudre ou comprimé, ou liquide comme une solution concentrée à diluer avant ou au moment de son utilisation ou une solution prête à l'emploi.

Le pH de la composition est compris entre 7,0 et 8,0, plus particulièrement entre 7,0 et 7,5.

Préférentiellement il s'agit d'une solution comprenant notamment des sels minéraux tels que des sels de magnésium et/ou de sélénium et/ou de zinc.

Selon un mode de réalisation particulier, la composition selon l'invention est une composition connue, utilisée pour la conservation de cellules, de tissus et/ou d'organes, à laquelle on ajoute au moins une cyclodextrine modifiée chargée positivement et du resvératrol et/ou un autre anti-oxydant adapté à la présente invention.

La composition selon l'invention peut être obtenue selon un procédé comprenant au moins :
- une étape d'association intime (interaction par tout type de liaison non covalente) d'une cyclodextrine modifiée chargée positivement avec un anti-oxydant, et
- une étape d'ajout du complexe formé au reste de la composition.

Selon un mode de réalisation, l'étape d'association intime entre la cyclodextrine et l'anti-oxydant peut être réalisée à partir de leur état naturel, par exemple un mélange solide poudre-poudre.

Préférentiellement, l'étape d'association intime de la cyclodextrine avec l'anti-oxydant, comprend les étapes suivantes :
- mise en solution de la cyclodextrine modifiée chargée positivement dans de l'eau,
- ajout de l'anti-oxydant dans cette solution,
- ajustement du pH entre 7 et 8,
- agitation, et
- éventuellement filtration de la solution suivie éventuellement d'une étape de séchage préférentiellement par lyophilisation.

L'étape d'ajout du mélange intime cyclodextrine/anti-oxydant au reste de la composition, peut consister à :
- l'ajouter à d'autres composés, notamment des sels minéraux tels que des sels de magnésium et/ou de sélénium et/ou de zinc, un imperméant, des nucléotides, etc. pour former une solution de conservation de cellules, tissus ou organes,ou
- l'introduire dans une solution connue de conservation de cellules, tissus ou organes, telle que par exemple ViaSpan®, SCOT®, Custodiol® ou IGL-1®.

Le complexe cyclodextrine/ anti-oxydant peut être ajouté sous sa forme solide après séchage ou sous sa forme liquide avant séchage.

Selon une variante, l'étape d'ajout du complexe au reste de la composition, peut consister à introduire l'association intime cyclodextrine/anti-oxydant dans un mélange pour fabriquer une solution destinée à être administrée par voie intraveineuse.

La composition selon l'invention comprenant au moins une cyclodextrine modifiée chargée positivement, préférentiellement associée à un anti-oxydant, peut être utilisée dans le cas d'une greffe en tant que produit thérapeutique annexe pour conserver des cellules, tissus ou organes entre le prélèvement sur un donneur et la greffe sur un receveur.

En particulier, elle peut être utilisée pour la transplantation d'organes en ischémie froide avant reperfusion.

Elle peut également être utilisée en thérapie cellulaire pour la conservation de cellules, par exemple de cellules souches, ou en thérapie tissulaire pour la conservation de tissus.

Selon un autre aspect, la composition selon l'invention est utile pour traiter un donneur ou un receveur d'organes, de tissus ou de cellules.

L'invention vise donc aussi son utilisation pour la fabrication d'un médicament destiné à pré-traiter un donneur ou un receveur d'organe, de tissu ou de cellules. Par ailleurs, la composition selon l'invention est également utile pour la fabrication d'un médicament, pour le post-traitement d'un receveur, destiné à être administré à une personne greffée pour prolonger la durée de vie du ou des greffons.

### I. Exemples de composition selon l'invention

Les exemples de composition selon l'invention sont des solutions constituées par un liquide de conservation d'organes ViaSpan® ou Celsior®, auquel on a ajouté :
- soit des cyclodextrines modifiées chargées positivement uniquement, à savoir la propane diamine-β-CD,
- soit un mélange intime propane diamine-β-CD/resvératrol.

Les résultats obtenus avec ces solutions sont ensuite comparés aux résultats obtenus avec une solution ViaSpan® seule (solution 1) ou Celsior® seule (solution 5).

### 1.1 Propane diamine-5-Cb seule / Viaspan®

Un procédé d'obtention consiste à reprendre 15,77 mg de propane diamine-β-CD en poudre dans 10 mL de milieu de conversation ViaSpan® pour obtenir une solution concentrée 100 fois. Cette solution est référencée solution 4 sur les figures.

Un autre exemple de solution selon l'invention peut être obtenu en reprenant 1577 mg de propane diamine-β-CD en poudre dans 10 mL de milieu de conversation ViaSpan®.

Avantageusement la poudre se dissout instantanément du fait du caractère très soluble de la cyclodextrine.

### I.2 Mélange intime propane diamine-β-CD/resvératrol / Viaspan®

Le procédé d'obtention consiste dans un premier temps à mélanger intimement des molécules de resvératrol (fournies par ONCOPHYT ou SHAANXI SCIPHAR Biotechnologies, Ltd ou ABATRA Technology co., Ltd) avec des molécules de propane diamine-β-CD (produite par BIOCYDEX), puis à introduire le mélange intime obtenu dans un liquide de conservation d'organes ViaSpan®.

La préparation du mélange intime s'effectue selon le mode opératoire suivant :
- on ajoute 4,57 mg de resvératrol à 9 mL d'une solution contenant 2495,3 mg de propane diamine-β-CD dans de l'eau,
- le pH est ajusté à 7,4,
- l'ensemble est agité pendant 5 à 24 heures à 24°C et à l'abri de la lumière,
- la solution est ensuite filtrée sur filtre PVDF hydrophile Millipore puis lyophilisée pour donner une poudre contenant un mélange intime dont le rapport molaire resvératrol/propane diamine-β-CD est de 1/109,
- le mélange intime lyophilisé est conservé dans des flacons ambrés à 4°C,
- 15,8 mg du mélange intime sont ensuite repris dans 10 mL du milieu de conservation ViaSpan® pour obtenir une solution concentrée 100 fois, référencée solution 2 sur les figures ; et en parallèle 1580 mg du mélange intime sont également repris dans 10 mL du milieu de conservation ViaSpan® pour obtenir une solution référencée solution 3 sur les figures.

Avantageusement, la poudre se dissout instantanément du fait du masquage du caractère hydrophobe du resvératrol par la cyclodextrine et du caractère très soluble de la cyclodextrine.

### I.3 Mélange intime propane diamine-β-Cb/resvératrol / Celsior®

Le procédé d'obtention consiste dans un premier temps à mélanger intimement des molécules de resvératrol (fournies par ONCOPHYT ou SHAANXI SCIPHAR Biotechnologies, Ltd ou ABATRA Technology co., Ltd) avec des molécules de propane diamine-β-CD (produite par BIOCYDEX), puis à introduire le mélange intime obtenu dans un liquide de conservation d'organes Celsior®.

La préparation du mélange intime s'effectue selon le mode opératoire suivant :
- on ajoute 4,57 mg de resvératrol à 9 mL d'une solution contenant 2495,3 mg de propane diamine-β-CD dans de l'eau,
- le pH est ajusté à 7,4,
- l'ensemble est agité pendant 5 à 24 heures à 24°C et à l'abri de la lumière,
- la solution est ensuite filtrée sur filtre PVDF hydrophile Millipore puis lyophilisée pour donner une poudre contenant un mélange intime dont le rapport molaire resvératrol/propane diamine-β-CD est de 1/109,
- le mélange intime lyophilisé est conservé dans des flacons ambrés à 4°C,
- 15,8 mg du mélange intime sont ensuite repris dans 10 mL du milieu de conservation Celsior® pour obtenir une solution concentrée 100 fois, référencée solution 6 sur les figures.

Avantageusement, la poudre se dissout instantanément du fait du masquage du caractère hydrophobe du resvératrol par la cyclodextrine et du caractère très soluble de la cyclodextrine.

### II. Effets de la composition selon l'invention.

La composition selon l'invention a été testée sur la conservation de reins destinés à la transplantation.

Le modèle le mieux adapté à l'étude in vivo des solutions de conservation, en ce qui concerne la greffe de rein, est l'autotransplantation rénale chez le porc.

Ce modèle consiste à transplanter chez le même animal le rein gauche, afin d'étudier les effets de la conservation et de la reperfusion sans avoir les problèmes de compatibilité liés à l'allotransplantation.

Le rein gauche est prélevé, conservé puis réimplanté. Lors de l'implantation du rein gauche, le rein droit est enlevé ce qui reproduit la situation clinique humaine, où il n'y a que le greffon qui soit susceptible d'assurer la fonction rénale.

Le protocole opératoire est décrit ci-après.

Le rein gauche est prélevé sur le porc : il est rincé puis conservé à 4°C avec une solution de conservation ViaSpan® seule (solution 1), solution de conservation Celsior® seule (solution 5), ou une des solutions telles qu'obtenues aux points I.1, I.2 ou I.3.

La durée de conservation est de 24 heures, temps de conservation nécessaire pour pouvoir mesurer une activité discriminante.

Au bout de 24 heures le rein est retransplanté et l'animal observé pour une période de 3 mois.

Les paramètres étudiés sont :
- la fonction rénale : urée et créatinine sérique et urinaire, ionogramme sanguin et urinaire et protidémie et protéinurie, et
- l'évolution tissulaire par une analyse histologique : prélèvement d'échantillons par biopsie rénale réalisée 24 heures, 7 jours et 1 mois après transplantation, étude morphologique de ces échantillons avec observation de l'aspect de nécrose tubulaire, de l'inflammation et de l'apparition d'atrophie tubulaire,
- le développement de la fibrose, l'inflammation et l'atrophie tubulaire après sacrifice de l'animal (3 mois après transplantation).

Les résultats concernant la reprise de la diurèse obtenus pour trois essais avec des solutions de Viaspan® seule ou des solutions présentées en I.1 et I.2, sont présentés en ml/24h dans le tableau suivant :

| Diurèse en mL.jour⁻¹ | J=0 | J=1 | J=3 | J=5 | J=7 | J=14 | J=30 | J=90 |
|---|---|---|---|---|---|---|---|---|
| ViaSpan® (solution 1) | 2100 | 200 | 2300 | 5767 | 7600 | 7600 | 7067 | 6500 |
| ViaSpan® + propane diamine-β-CD + resvératrol, 15,8 mg/L (solution 2) | 2033 | 2100 | 6767 | 5633 | 6133 | 4267 | 5733 | 3650 |
| ViaSpan® + propane diamine-β-CD + resvératrol, 1580 mg/L (solution 3) | 2733 | 1367 | 1900 | 2767 | 2033 | 2867 | 2900 | / |
| ViaSpan® + propane diamine-β-CD, 15,77 mg/L (solution 4) | 2300 | 567 | 2967 | 925 | 1767 | 1567 | 2367 | / |
| ViaSpan® + propane diamine-β-CD, 1577 mg/L | 2433 | 950 | 2300 | 550 | 2017 | 2733 | 5000 | / |

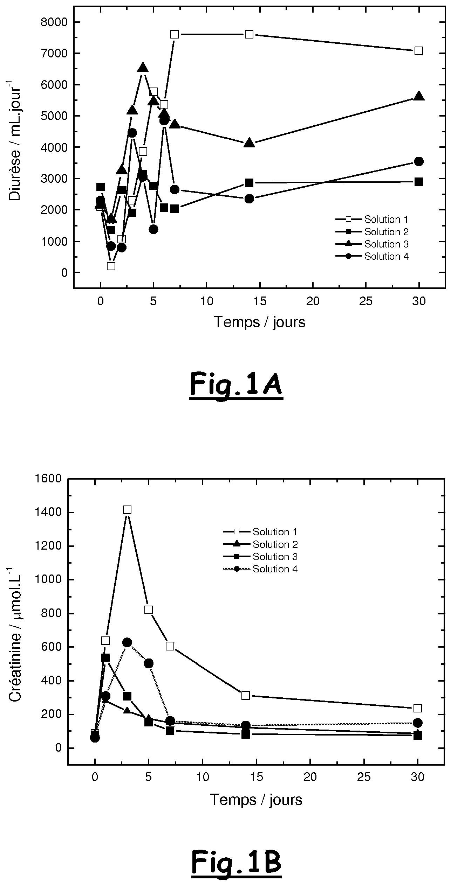

### Ces résultats sont en partie représentés sur la figure 1A.

On constate que l'utilisation des compositions selon l'invention permet d'améliorer considérablement la reprise de diurèse par rapport à la solution de référence ViaSpan®.

On observe également que le profil de la poly-urie consécutive à la conservation et la transplantation est mieux maîtrisé lors de l'utilisation des compositions selon l'invention. La reconstitution de l'urémie est également plus rapide.

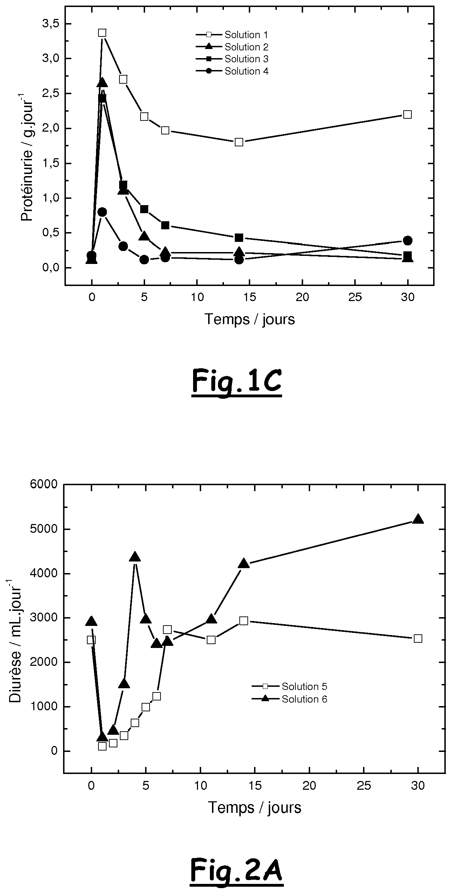

Les résultats concernant la reprise de la diurèse obtenus pour trois essais avec des solutions de Celsior® seule (solution 5) ou de la solution de I.3 (solution 6), sont présentés sur la figure 2A. On constate également que l'utilisation d'une composition selon l'invention permet d'améliorer considérablement la reprise de diurèse par rapport à la solution Celsior®.

Concernant la créatinine plasmatique, les résultats obtenus avec les solutions Viaspan® supplémentées ou non de compositions selon l'invention sont regroupés dans le tableau qui suit.

| Créatinine en µmol.L⁻¹ | J=0 | J=1 | J=3 | J=5 | J=7 | J=14 | J=30 | J=90 |
|---|---|---|---|---|---|---|---|---|
| ViaSpan® (solution 1) | 85 | 637 | 1416 | 823 | 605 | 312 | 235 | 207 |
| ViaSpan® + propane diamine-β-CD + resvératrol, 15,8 mg/L (solution 2) | 85 | 280 | 220 | 174 | 150 | 122 | 85 | 87 |
| ViaSpan® + propane diamine-β-CD + resvératrol, 1580 mg/L (solution 3) | 77 | 535 | 308 | 153 | 102 | 82 | 76 | / |
| ViaSpan® + propane diamine-β-CD, 15,77 mg/L (solution 4) | 62 | 309 | 627 | 503 | 160 | 133 | 149 | 120 |
| ViaSpan® + propane diamine-β-CD, 1577 mg/L | 73 | 575 | 836 | 906 | 754 | 294 | 163 | / |

Ces résultats sont en partie représentés sur la figure 1B.

On constate que les compositions selon l'invention permettent de diminuer efficacement la créatinine plasmatique après transplantation du rein comparativement à la solution de référence ViaSpan®.

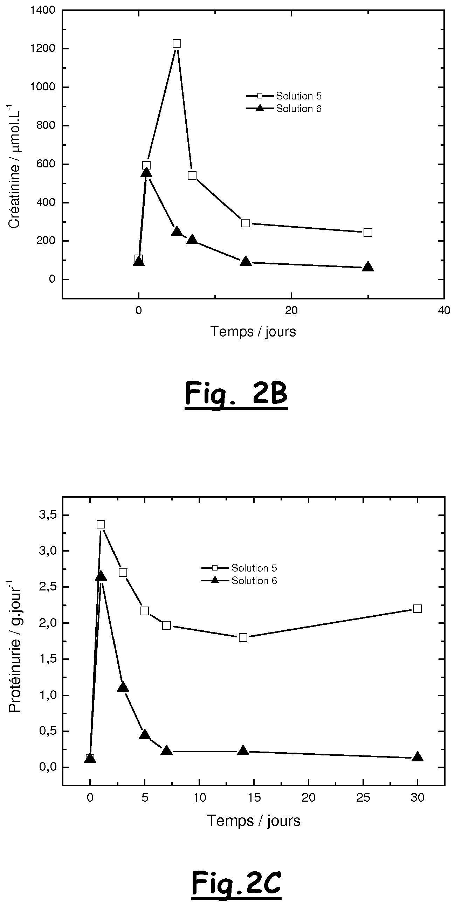

Les résultats concernant la créatinine plasmatique obtenus pour trois essais avec des solutions de Celsior® seule (solution 5) ou de la solution de I.3 (solution 6), sont présentés sur la figure 2B.

On constate également que les compositions selon l'invention permettent de diminuer efficacement la créatinine plasmatique après transplantation du rein comparativement à la solution Celsior®.

Concernant la protéinurie, les résultats obtenus avec les solutions Viaspan® supplémentées ou non de compositions selon l'invention sont regroupés dans le tableau suivant :

| Protéinurie en g.jour⁻¹ | J=0 | J=1 | J=3 | J=5 | J=7 | J=14 | J=30 | J=90 |
|---|---|---|---|---|---|---|---|---|
| ViaSpan® (solution 1) | 0,12 | 3,37 | 2,70 | 2,17 | 1,97 | 1,60 | 2,20 | 2,53 |
| ViaSpan® + propane diamine-β-CD + resvératrol, 15,8 mg/L (solution 2) | 0,11 | 2,64 | 1,1 | 0,44 | 0,22 | 0,22 | 0,13 | 0,15 |
| ViaSpan® + propane diamine-β-CD + resvératrol, 1580 mg/L (solution 3) | 0,12 | 2,43 | 1,19 | 0,84 | 0,61 | 0,43 | 0,18 | / |
| ViaSpan® + propane diamine-β-CD, 15,77 mg/L (solution 4) | 0,18 | 0,80 | 0,31 | 0,12 | 0,15 | 0,12 | 0,39 | 0,29 |
| ViaSpan® + propane diamine-β-CD, 1577 mg/L | 0,12 | 0,89 | 0,27 | 0,68 | 0,36 | 0,35 | 0,31 | / |

Ces résultats sont en partie représentés sur la figure 1C.

Là encore on constate que l'utilisation des compositions selon l'invention permet de considérablement mieux maîtriser les taux de protéinurie.

Par ailleurs, l'étude histologique montre que l'utilisation des compositions selon l'invention réduit significativement l'expression du Complexe Majeur d'Histocompatibilité (CMH) de type II et diminue les marqueurs d'apoptose et de fibrose.

Les résultats concernant la protéinurie obtenus pour trois essais avec des solutions de Celsior® seule (solution 5) ou de la solution de I.3 (solution 6), sont présentés sur la figure 2C.

On constate également que l'utilisation des compositions selon l'invention permet de considérablement mieux maîtriser les taux de protéinurie par rapport à l'utilisation de Celsior® seule.

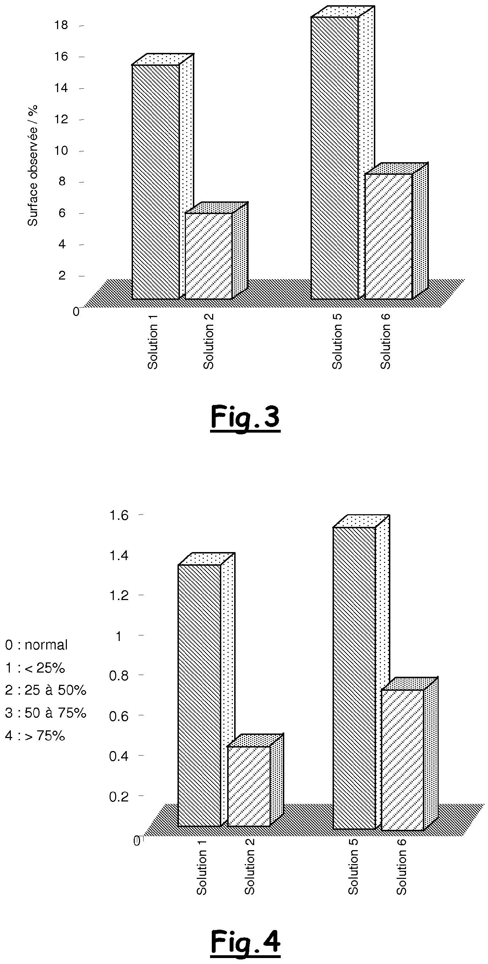

Selon un autre aspect, l'analyse anatomopathologique des organes 30 jours après la greffe montre une nette diminution des surfaces fibrosées en présence de compositions selon l'invention (figure 3) ainsi qu'une très nette diminution de l'atrophie tubulaire (figure 4) par rapport aux solutions de référence Viaspan® et Celsior®.

Ces résultats sont confirmés après sacrifice des animaux 90 jours après la greffe, avec de très faibles surfaces fibrosées et l'absence d'atrophie tubulaire. De même l'inflammation lymphoïde, présente dans le cas de traitement avec une solution commerciale de référence, apparaît très faible en présence d'une solution selon l'invention.

Bien entendu, l'invention n'est évidemment pas limitée aux exemples représentés et décrits ci-dessus.

## Revendications

1. Composition pour protéger et/ou préserver des cellules, des tissus ou des organes, **caractérisée en ce qu'**elle présente un pH compris entre 7 et 8 et **en ce qu'**elle comprend au moins une cyclodextrine modifiée, chargée positivement.

2. Composition pour protéger et/ou préserver des cellules, des tissus ou des organes selon la revendication 1, **caractérisée en ce que** ladite cyclodextrine répond à la formule : 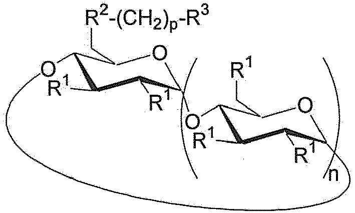 dans laquelle
- R¹ représente OH ou R²-(CH₂)p-R³ ;
- R² représente -O- ou -S- ou -NH- ou -NR⁴;
- R³ représente -OH ou -NH₂ ou -NHR⁴;
- R⁴ représente un groupe allyle ;
- n est égal à 5, 6 ou 7, et p est un nombre entier allant de 2 à 10 ;
les p, R², R³ et R⁴ pouvant être différents lorsque un ou plusieurs des R¹ représentent R²-(CH₂)p-R³, et, lorsque R² et R³ représentent respectivement -NR⁴- et NHR⁴, avec R⁴ étant un groupe alkyle, celui-ci est de préférence un groupe alkyle inférieur, ayant de 1 à 4 atomes de carbone.

3. Composition pour protéger et/ou préserver des cellules, des tissus ou des organes selon la revendication 1 ou 2, **caractérisée en ce qu'**elle comprend au moins un mélange intime cyclodextrine-antioxydant.

4. Composition pour protéger et/ou préserver des cellules, des tissus ou des organes selon la revendication 3, **caractérisée en ce qu'**elle comprend un ratio molaire anti-oxydant/cyclodextrine compris entre 1/1,5 et 1/150.

5. Composition pour protéger et/ou préserver des cellules, des tissus ou des organes selon l'une des revendications 3 ou 4, **caractérisée en ce que** l'anti-oxydant est le resvératrol.

6. Composition pour protéger et/ou préserver des cellules, des tissus ou des organes selon l'une des revendications 3 ou 4, **caractérisée en ce que** l'anti-oxydant est la curcumine.

7. Composition pour protéger et/ou préserver des cellules, des tissus ou des organes selon l'une quelconque des précédentes revendications, **caractérisée en ce que** la cyclodextrine est choisie parmi les cyclodextrines mono modifiées.

8. Composition pour protéger et/ou préserver des cellules, des tissus ou des organes selon l'une quelconque des précédentes revendications, **caractérisée en ce que** la cyclodextrine est la 6A-(3-Aminopropylamino)-6A-desoxy-cyclomatoheptaose ou la 6A-(2-Aminoethylamino)-6A-desoxy-cyclomaltoheptaose.

9. Utilisation d'une composition selon l'une quelconque des revendications 1 à 8, pour protéger, préserver et/ou conserver des organes ex vivo, en particulier en ischémie froide avant reperfusion.

10. Utilisation d'une composition selon l'une quelconque des revendications 1 à 8, pour protéger, préserver et/ou conserver des tissus ou des cellules ex vivo.

11. Utilisation d'une composition selon l'une quelconque des revendications 1 à 8, pour la fabrication d'un médicament destiné au prétraitement d'un donneur et/ou d'un receveur d'organe.

12. Utilisation d'une composition selon l'une quelconque des revendications 1 à 8, pour la fabrication d'un médicament destiné à être administré à une personne greffée pour prolonger la durée de vie du ou des greffon(s).

## Patentansprüche

1. Zusammensetzung zum Schutz und/oder Erhalt von Zellen, Geweben oder Organen, **dadurch gekennzeichnet, dass** diese einen pH zwischen 7 und 8 aufweist und dass diese wenigstens ein positiv geladenes modifiziertes Cyclodextrin aufweist.

2. Zusammensetzung zum Schutz und/oder Erhalt von Zellen, Geweben oder Organen nach Anspruch 1, **dadurch gekennzeichnet, dass** das Cyclodextrin der Formel entspricht: 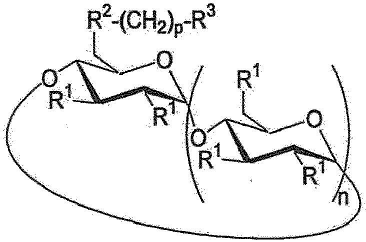 für die gilt:
- R¹ steht für OH oder R²-(CH₂)p-R³;
- R² steht für -O- oder -S- oder -NH- oder -NR⁴;
- R³ steht für -OH oder -NH₂ oder -NHR⁴;
- R⁴ steht für eine Alkylgruppe;
- n ist gleich 5, 6 oder 7, und p ist eine ganze Zahl die einen Wert von 2 bis 10 annehmen kann;
wobei die p, R², R³ und R⁴ verschieden sein können, wenn ein oder mehrere der R¹ für R²-(CH₂)p-R³ steht oder stehen, und wobei, wenn R² und R³ jeweils für -NR⁴- und NHR⁴ stehen und R⁴ eine Alkylgruppe ist, diese vorzugsweise eine kurze Alkylgruppe mit 1 bis 4 Kohlenstoffatomen ist.

3. Zusammensetzung zum Schutz und/oder Erhalt von Zellen, Geweben oder Organen nach Anspruch 1 oder 2, **dadurch gekennzeichnet, dass** diese wenigstens eine gute Mischung von Cyclodextrin und einem Antioxidans aufweist.

4. Zusammensetzung zum Schutz und/oder Erhalt von Zellen, Geweben oder Organen nach Anspruch 3, **dadurch gekennzeichnet, dass** diese ein molares Verhältnis von Antioxidans zu Cyclodextrin zwischen 1/1,5 und1/150 aufweist.

5. Zusammensetzung zum Schutz und/oder Erhalt von Zellen, Geweben oder Organen nach einem der Ansprüche 3 oder 4, **dadurch gekennzeichnet, dass** das Antioxidans Resveratrol ist.

6. Zusammensetzung zum Schutz und/oder Erhalt von Zellen, Geweben oder Organen nach einem der Ansprüche 3 oder 4, **dadurch gekennzeichnet, dass** das Antioxidans Curcumin ist.

7. Zusammensetzung zum Schutz und/oder Erhalt von Zellen, Geweben oder Organen nach einem der vorhergehenden Ansprüche, **dadurch gekennzeichnet, dass** das Cyclodextrin aus mono-modifizierten Cyclodextrinen ausgewählt ist.

8. Zusammensetzung zum Schutz und/oder Erhalt von Zellen, Geweben oder Organen nach einem der vorhergehenden Ansprüche, **dadurch gekennzeichnet, dass** das Cyclodextrin 6A-(3-Aminopropylamino)-6A-desoxy-cyclomatoheptaose oder 6A-(2-Aminoethylamino)-6A-desoxy-cyclomaltoheptaose ist.

9. Verwendung einer Zusammensetzung nach einem der Ansprüche 1 bis 8 zum Schutz, Erhalt und/oder Konservieren von Organen ex vivo, insbesondere in kalter Ischämie vor einer Reperfusion.

10. Verwendung einer Zusammensetzung nach einem der Ansprüche 1 bis 8 zum Schutz, Erhalt und/oder Konservieren von Geweben oder Zellen ex vivo.

11. Verwendung einer Zusammensetzung nach einem der Ansprüche 1 bis 8, für die Herstellung eines Medikaments das für die Vorbehandlung eines Organspenders und/oder eines Organempfängers bestimmt ist.

12. Verwendung einer Zusammensetzung nach einem der Ansprüche 1 bis 8, für die Herstellung eines Medikaments, das dazu bestimmt ist, einer transplantierten Person verabreicht zu werden, um die Lebensdauer des Transplantats oder der Transplantate zu verlängern.

## Claims

1. A composition for protecting and/or preserving cells, tissues or organs, **characterised in that** it has a pH of between 7 and 8 and **in that** it comprises at least one positively charged modified cyclodextrin.

2. A composition for protecting and/or preserving cells, tissues or organs according to claim 1, **characterised in that** said cyclodextrin complies with the formula: 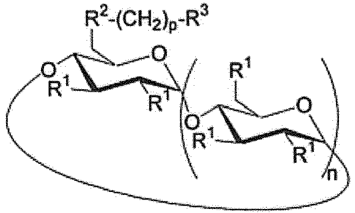 in which
- R¹ represents OH or R²-(CH₂)p-R³;
- R² represents -O- or -S- or -NH- or -NR⁴;
- R³ represents -OH or -NH₂ or -NHR⁴;
- R⁴ represents an alkyl group;
- n is equal to 5, 6 or 7, and p is an integer number ranging from 2 to 10;
the ps, R²s, R³s and R⁴s being able to be different when one or more or the R¹s represents R²-(CH₂)p-R³, and, when R² and R³ represent respectively -NR⁴- and NHR⁴, with R⁴ being an alkyl group, the latter being preferably a lower alkyl group, having 1 to 4 carbon atoms.

3. A composition for protecting and/or preserving cells, tissues or organs according to claim 1 or 2, **characterised in that** it comprises at least one intimate cyclodextrin/antioxidant mixture.

4. A composition for protecting and/or preserving cells, tissues or organs according to claim 3, **characterised in that** it comprises an antioxidant/cyclodextrin molar ratio of between 1/1.5 and 1/150.

5. A composition for protecting and/or preserving cells, tissues or organs according to one of claims 3 or 4, **characterised in that** the antioxidant is resveratrol.

6. A composition for protecting and/or preserving cells, tissues or organs according to one of claims 3 or 4, **characterised in that** the antioxidant is curcumin.

7. A composition for protecting and/or preserving cells, tissues or organs according to any of the preceding claims, **characterised in that** the cyclodextrin is chosen from monomodified cyclodextrins.

8. A composition for protecting and/or preserving cells, tissues or organs according to any of the preceding claims, **characterised in that** the cyclodextrin is 6A-(3-aminopropylamino)-6A-deoxy-cyclomaltoheptaose or 6A-(2-aminoethylamino)-6A-deoxy-cyclomaltoheptaose.

9. Use of a composition according to any of claims 1 to 8, for protecting, preserving and/or storing organs ex *vivo,* in particular in cold ischaemia before reperfusion.

10. Use of a composition according to any of claims 1 to 8, for protecting, preserving and/or storing tissues or cells ex *vivo.*

11. Use of a composition according to any of claims 1 to 8, for manufacturing a medication intended for the pretreatment of an organ donor and/or recipient.

12. Use of a composition according to any of claims 1 to 8, for manufacturing a medication intended to be administered to a person who has received a graft in order to prolong the life of the graft or grafts.
